# EUROPÄISCHE PATENTSCHRIFT

(11) **EP 0 618 216 B1**
(45) Veröffentlichungstag und Bekanntmachung des Hinweises auf die Patenterteilung: **10.09.1997**
(21) Anmeldenummer: 94104472.9
(22) Anmeldetag: 22.03.1994
(51) Int. Cl.: C07H 15/04, C11D 1/42, C11D 1/645, A61K 7/00

(54) **1-N-Alkylaminoisomalt, ihre Herstellung aus Isomaltulose sowie ihre Verwendung als oberflächenaktive Mittel**
N-alkylisomaltamines, their preparation from isomaltulose and their application as surfactants
N-alkyl-isomaltamines, méthode de préparation à partir de l'isomaltulose et leur utilisation comme agents tensioactifs

(30) Priorität: 27.03.1993 DE 4310032
(43) Veröffentlichungstag der Anmeldung: 05.10.1994
(73) Patentinhaber: SÜDZUCKER AKTIENGESELLSCHAFT MANNHEIM/OCHSENFURT, D-68165 Mannheim (DE)
(72) Erfinder: Kunz, Markwart, Dr., D-38104 Braunschweig (DE); Guderjahn, Lutz, D-38106 Braunschweig (DE); Schüttenhelm, Matthias, D-38114 Braunschweig (DE); Kowalczyk, Jörg, Dr., D-67269 Grünstadt (DE)
(74) Vertreter: Gleiss & Grosse

(56) Entgegenhaltungen:
- EP-A- 0 220 676
- EP-A- 0 255 033
- EP-A- 0 383 023
- EP-A- 0 383 024
- WO-A-92/06984
- US-A- 2 181 929
- US-A- 2 838 487
- J. EXP. MED. Bd. 154 , 1981 Seiten 432 - 449 C. WOOD, E. KABAT 'Immunochemical Studies of Conjugates of Isomaltosyl Oligosaccharides to Lipid' & R.S. SHALLENBERGER 'ADVANCED SUGAR CHEMISTRY' 1982 , ELLIS HORWOOD LTD PUBLISHERS
- DATABASE WPI DERWENT PUBLICATIONS LTD., LONDON, GB AN 93-191473 (24) 'Novel amino sugar cpds.' & JP-A-5 117 216 (KAO CORP.) 14. Mai 1993
- MACROMOL. CHEM. Bd. 191 , 1990 Seiten 517 - 528 J. KLEIN 'New surfactant polymers based on carbohydrates'
- J. EXP. MED. Bd. 154, 1981, Seiten 432-449, C. WOOD et al.
- DATABASE WPI DERWENT PUBLICATIONS LTD., London, GB, AN 93-191473 (24)
- MACROMOL. CHEM. Bd. 191, 1990, Seiten 517-528, J. KLEIN

## Beschreibung

Gegenstand der Erfindung sind neuartige Produkte, die auf Basis des Rohstoffes Isomaltulose durch reduktive Aminierung mit längerkettigen Alkylaminen bzw. Alkylaminmischungen hergestellt werden. Die Verbindungen zeigen hervorragende oberflächenaktive Eigenschaften und eignen sich entsprechend ihrem HLB-Wert (hydrophilic, lipophilic-balance) als hydrophile Netzmittel, Emulgatoren, waschaktive Substanzen sowie als oberflächenaktive Neutralisationsmittel von anionischenTensiden.

Die reduktive Aminierung als Schlüsselreaktion zur Herstellung von Monoaminopolyolen ist bereits beschrieben, jedoch werden als Aminierungsmittel z. B. bei Glucose vorwiegend Ammoniak (US 2.016.962) und Hydrazin (US. 2.830.983) eingesetzt.

Auch die reduktive Aminierung von Isomaltulose mit Ammoniak und Hydrazin sowie in organischen Lösungsmitteln mit Natriumborhydriden ist bekannt. Isomaltulose, bei der es sich um ein 2-Keto-Disaccharid handelt, liefert bei der Reaktion die erwarteten korrespondierenden 2-Aminopolyole (DE 39 04 246).

Die reduktive Aminierung von reduzierenden Sacchariden mit Alkylaminen wurde ebenfalls am Beispiel Glucose (DD 13746) und Maltose (US 2.181.929) durchgeführt. Von Isomaltulose wird die Umsetzung mit N-Hexylamin beschrieben (DE 3904246). Gemäß diesen Untersuchungen wird bei der reduktiven Aminierung die Aminoalkylfunktion entsprechend der Position der Aldehyd- bzw. Ketofunktion im Saccharid eingeführt.

Führt man die reduktive Aminierung bei Isomaltulose jedoch mit längerkettigen N-Alkylaminen (C₈ bis C₂₁) durch, so erhält man nicht die 2-Aminoalkyl- sondern überraschenderweise die 1-Amino-alkylpolyole in hohen Ausbeuten. Versuche mit unterschiedlichen Aminen bestätigen, daß nur bei Aminen mit kürzeren Alkylkettenlängen Derivate gebildet werden, die auch in 2-Position entsprechend substituiert sind. Bei Alkylkettenlängen ab C₈ werden nur 1-substituierte Produkte beobachtet.

Die reduktive Aminierung von Isomaltulose mit z. B. N-Dodecylamin liefert in hohen Ausbeuten 1-N-Dodecylaminoisomalt entsprechend Formel I. Die Bezeichung 1-N-Dodecylaminoisomalt wurde der Einfachheit halber gewählt und beschreibt ein Gemisch aus 1-N-Dodecylamino-1-desoxy-6-O-(α-D-glucopyranosyl)-D-sorbit und 1-N-Dodecylamino-1-desoxy-6-O-(α-D-glucopyranosyl)-D-mannit. Die Herstellung des Sorbit-Derivates ist beschrieben ( C. Wood, E.A. Kabat, J. Exp. Med., 154 (2), 432-49 (1981)), jedoch wird hier die Verbindung aus Isomaltose hergestellt.

Die korrespondierende 1-N-Dodecylamino-1-desoxy-6-O-(α-D-glucopyranosyl)-D-mannit-Verbindung konnte NMR-spektroskopisch im Gemisch nachgewiesen werden und ist in der Literatur noch nicht beschrieben.

Neben diesen Verbindungen entsteht durch Spaltung auch das um 3 Kohlenstoffatome kettenverkürzte 1-N-Dodecylamino-1-desoxy-3-O-(α-D-glucopyranosyl)-glycerin. Besonders vorteilhaft hat sich der Einsatz einer höheren stöchiometrischen Menge (1,5 bis 6 Mol) Isomaltulose erwiesen. Auf diese Weise wird das N-Alkylamin fast vollständig umgesetzt, so daß aufgrund von Spaltungsreaktionen nur ein Gemisch aus den folgenden N-Alkylaminopolyolen entsteht, das praktisch frei an N-Alkylamin (< 0,5 %) ist.
- 1-N-Dodecylaminoisomalt
- 1-N-Dodecylamino-1-desoxy-sorbit
- 1-N-Dodecylamino-1-desoxy-3-O-(α-D-glucopyranosyl)-glycerin
- 1-N-Dodecylamino-1-desoxy-glycerin

Die reduktive Aminierung erfolgt:
a) In Gegenwart von Katalysatoren unter Verwendung von Wasserstoff in wäßrig/alkoholischer Lösung.
   Als Katalysatoren eignen sich für die Herstellung der erfindungsgemäßen Produkte und Mischungen Edelmetallträger-, Ganzmetallträger- sowie Mischmetallkatalysatoren, die entsprechend dem Stand der Technik bei Hydrierungen eingesetzt werden. Vorteilhaft hat sich hierbei die absatzweise Herstellung der Produkte bei Temperaturen von 40 bis 110 °C, einem Wasserstoffpartialdruck von 50 bis 300 bar und Verwendung von Raney-Nickel-Suspensionen im Slurryverfahren erwiesen.
b) Die Umsetzung kann jedoch auch in wäßriger/alkoholischer Lösung mit einem Festbettkatalysator in einem kontinuierlichen Verfahren realisiert werden. Nach einer Vorrührphase (0,1 bis 16 h) wird die reduktive Aminierung bei Temperaturen von 40 bis 110 °C, einer Isomaltulosekonzentration von 5 bis 40 Gew.-% und einem Wasserstoffpartialdruck von 50 bis 300 bar an einem Raney-Nickel-Festbettkontakt durchgeführt.
c) Als weitere Möglichkeit kann die Umsetzung auch in organischen Lösungsmitteln, z.B. in Dimethylformamid, im folgendem DMF genannt, realisiert werden. Diese Möglichkeit ist jedoch in technologischer Hinischt von untergeordneter Bedeutung, da der reduktive Abbau zum 1-N-Alkylamino-1-desoxy-glycerin der Formel II dabei zunimmt.

Außerordentlich gut hat sich die Zugabe von 0,5 bis 10 Gew.-% 1-N-Alkylamino-isomalt bzw. der Alkylpolyolmischung bewährt. Durch diese vorherige Produktzugabe kann eine bessere Homogenität der Reaktionslösung erreicht werden, so daß bei der katalytischen Reduktion eine höhere Reaktionsgeschwindigkeit beobachtet wird.

Bei der Zugabe einer höheren stöchiometrischen Menge an Isomaltulose und Produktzugabe führt die reduktive Aminierung zu einem Produktgemisch, das fast kein N-Alkylamin mehr enthält (unter 0,5 %). Die Abtrennung des N-Alkylamin aus dem Rohprodukt wird daher durch die erheblich einfachere Abtrennung der neutralen Polyole ersetzt.

Die resultierenden Produkte können zum einen mit dem noch im Gemisch befindlichen Zuckeralkohol (Isomalt, Mischung aus 6-O-(α-D-Glucopyranosyl-D-mannit bzw. -sorbit)) durch Verdampfung des Lösungsmittels direkt isoliert werden. Der Zuckeralkohol wird hier als ökologisch vertretbarer, biologisch leicht abbaubarer Füllstoff angesehen.

Die Zuckeralkohole können jedoch auch abgetrennt und beispielsweise als Hilfsstoffe in der Kunststoffindustrie eingesetzt werden.

Die aminierten N-Alkylaminopolyole können jedoch auch durch chromatographische Verfahren entweder als Gemisch bzw. einzeln isoliert werden. Die Trennung solcher Produkte erfolgt an einer Kationenaustauschersäule, die in der entsprechenden Ammoniumform vorliegt.

Nach Abtrennung der nicht aminierten Bestandteile (Zuckeralkohol) erfolgt die Elution der Produkte mit Ammoniaklösung.

Ein weiteres technisch interessantes Verfahren zur Gewinnung der aminierten Produkte aus dem Zuckeralkohol/N-Alkylaminopolyol-Gemisch konnte durch Elektrodialyse realisiert werden.

Durch Anlegen des elektrischen Feldes werden die potentiell kationischen N-Alkylpolyole aus dem Diluat ins Konzentrat überführt. Dieses Verfahren führt zu noch höheren Elektrodialysegeschwindigkeiten, wenn eine Begasung des Diluates mit CO₂ erfolgt oder Ansäuern mit einer Säure erfolgt. Hierbei werden die N-Alkylaminopolyole als Salze in das Konzentrat überführt und können anschließend durch Entfernung des Lösungsmittels gewonnen werden.

Entsprechend ihres Eigenschaftsprofiles können die Produkte bzw. die Produktmischungen als hydrophile Netzmittel, Emulgatoren oder waschaktive Substanzen eingesetzt werden.

In Tabelle 2 sind die Oberflächenspannungen σ einer Reihe von Verbindungen sowie deren Mischungen angegeben. Die Oberflächenspannungen wäßriger Lösungen der Substanzen liegen deutlich unter 30 mN/m, so daß die Produkte im Detergentienbereich eingesetzt werden können.

Besonders als Tenside geeignet sind die Produkte aufgrund ihres ökologischen Verhaltens. Durch den Zuckerbaustein, der ca. 60 % des Gesamtmoleküls ausmacht, zeigen die Produkte in OECD-Screening-Tests (Organisation for Economic Co-Operation and Development; s. auch Verordnung über die Abbaubarkeit anionischer und nichtionischer grenzflächenaktiver Stoffe in Wasch- und Reinigungsmitteln vom 30.Januar 1977 (BGBl. I, S. 244)) eine gute Abbaubarkeit und können als gut biologisch abbaubar eingestuft werden.

Ein weiterer Vorteil der Verbindungen ist die sehr niedrige kritische Micellbildungskonzentration, kurz CMC genannt. Produkte mit niedriger CMC werden als besonders hautschonend eingestuft und können im kosmetischen Sektor als Emulgatoren Anwendung finden.

Ein weiterer Vorteil der erfindungsgemäßen Produkte ist, daß sie im gesamten pH-Bereich auch als entsprechende N-Alkylaminopolyolmischung (bis zu einer mittleren Alkylkettenlänge bis C₁₄) wasserlöslich sind, wodurch sie sich von bekannten Alkylaminomonosacchariden unterscheiden.

Auch bei Gegenwart eines anionischen Tensides, z. B. Fettalkoholethersulfaten bzw. Sulfonaten bleiben die Produkte in Lösung. Durch diese Eigenschaft verhindern sie ein Absetzen von flockenden Anteilen auf Wäsche bzw. Fasern und fungieren ähnlich wie kommerzielle Weichspüler (z. B. quartäre Ammoniumverbindungen). In der neueren Literatur werden diese bereits beim Waschvorgang als Weichspüler wirkenden Stoffe als "Softergents" bezeichnet. Die erfindungsgemäßen Produkte bzw. Produktgemische können in diese Verbindungsklasse ebenfalls eingestuft werden.

Der potentielle Einsatzbereich dieser neuen Produkte liegt aufgrund des Eigenschaftsprofils im Bereich Waschmittel, kosmetische Produkte wie z. B. Haarshampoo, Duschgele, Cremes. Als Emulgatoren können sie in technischen Bereichen wie z. B. Spülmittel sowie als Emulgatoren für organische Medien dienen. Ein Einsatz dieser Verbindungen ist sicherlich auch im Bereich der tertiären Erdölförderung, z. B. als Flutungshilfsmittel zu finden.

**Im folgenden wird die Erfindung an Hand von Beispielen näher erläutert.**

### Beispiel 1

### Herstellung von 1-N-Dodecylaminoisomalt durch reduktive Aminierung

50 g Isomaltulose (0,139 mol) werden in 180 ml VE-Wasser und 57 ml 2-Propanol gelöst und diese Lösung wird in einem temperierbaren Reaktor auf 10 °C abgekühlt. Anschließend tropft man eine Lösung aus 7,37 g N-Dodecylamin (0,0397 mol) in 120 ml VE-Wasser und 69 ml 2-Propanol dazu und rührt 30 Minuten weiter.

Das Reaktionsgemisch wird in einen Autoklaven gefüllt, 24 g Raney-Nickel-Suspension (Feuchtmasse) zugesetzt und bei 150 bar Wasserstoffpartialdruck, 50 °C und einer Rührgeschwindigkeit von 1 000 U/min 24 Stunden lang hydriert. Nach Abtrennung des Katalysates wird am Rotationsverdampfer ein geengt und das verbleibende Reaktionsgemisch chromatographisch aufgearbeitet.

Die Trennung der erhaltenen 1-N-Dodecylpolyole vom Zuckeralkohol erfolgt an einer Kationenaustauschersäule (Firma Serva, Amberlite IR-120, NH₄-Form). Die Lösung wird auf die Säule (Bettvolumen 1,7 l) aufgegeben und man wäscht mit dem 5fachen Bettvolumen Wasser/2-Propanol (90/10 ; v/v) nach. Hierbei wird der Zuckeralkohol entfernt.
Die Elution der 1-N-Alkylpolyole vom Kationenaustauscherharz erfolgt mit dem 5fachen Bettvolumen 10 % NH₃-Lösung/2-Propanol (50/50; v/v).
Das Eluat wird einrotiert, NH₃ und Propanol entfernt und die Produkte werden anschließend durch Gefriertrocknung isoliert. Ausbeute (bezogen auf eingesetztes N-Dodecylamin): 98 %. Das Produktspektrum ist in Tabelle 1 aufgeführt.

### Beispiel 2

### Reduktive Aminierung (kontinuierliches Verfahren)

Die Isomaltulose und Dodecylaminlösung (Zusammensetzung wie unter Beispiel 1 beschrieben) sowie Wasserstoff werden über einen Mischer durch einen mit Festbett-Raney-Nickel gefüllten Rohreaktor (500 ml) kontinuierlich gepumpt. Die Hydrierung erfolt bei einem Druck von 150 bar, einer Temperatur von 70 °C und einer Pumpgeschwindigkeit von 0,1 l/h.

Nach Einstellung des Gleichgewichtes wird ein Produkt entnommen, das nach der Aufarbeitung (siehe Beispiel 1) eine ähnliche Zusammensetzung hat wie bei der absatzweisen Hydrierung im Suspensionsverfahren.

### Beispiel 3

### Reduktive Aminierung in Dimethylformamid (DMF) als Lösungsmittel

Die reduktive Aminierung von Isomaltulose und Dodecylamin (molares Verhältnis 3:1) wird in DMF entsprechend der im Beispiel 1 beschriebenen Bedingungen hydriert. Gegenüber der Umsetzung in wäßrigem Alkohol variiert die Produktzusammensetzung.

In Tabelle I sind die Produktzusammensetzungen der Hydrierungen in DMF und in Wasser/Isopropanol sowie das Vergleichsbeispiel 1 mit einer kürzeren Alkylkettenlänge (mit Butylamin) angegeben. Es geht hieraus hervor, daß auch in DMF bei längeren Alkylketten nur die 1-N-Alkylaminoverbindung gebildet wird.

**Tabelle I:**

| Die folgende Tabelle I zeigt die Produktzusammensetzungen von Hydrierungen in DMF und in Wasser/Isopropanol sowie das Vergleichsbeispiel 1 mit einer kürzeren Alkylkettenlänge mit den mittels HPLC-Systems ermittelten normierten Retentionszeiten als Rf-Werte ausgedrückt. | | | | | |
|---|---|---|---|---|---|
| Ausbeute [Mol] (bezogen auf einges. Alkylamin) | | | | | |
| | in DMF | in Wasser/Isopropanol | | Rf-Wert in H₂O | |
| *Aminierte Produkte* | *R = Dodecyl* | *R = Butyl* | *R = Dodecyl* | *R = Butyl* | *R = Dodecyl* |
| 1-N-Alkylaminoisomalt | 31,5 | 47 | 63 | 4,7 | 3,4 |
| 2-N-Alkylaminoisomalt | - | 32 | 0 | 5,2 | - |
| 1-N-Alkylamino-1-desoxy-3-glucosylglycerin | 64,7 | 5,0 | 24,8 | 2,6 | 3,7 |
| 1-N-Alkylamino-1-desoxy-sorbit/mannit | 3,8 | 5,0 | 8,4 | 4,6 | 5,8 |

### Beispiel 4

### Reduktive Aminierung mit Produktzusatz

Ein Teil des Produktes aus Beispiel 1 (5% Produktlösung) wurde wieder mit frischer Eduktlösung zurück in den Reaktor eingefüllt. Unter gleicher Parametereinstellung konnte die reduktive Aminierung bereits nach 16 Stunden abgebrochen werden. Ausbeute (bezogen auf 1-N-Dodecylamin): > 98 %

### Beispiel 5

### Gewinnung der N-Alkylaminopolyole aus dem Reaktionsgemisch mittels Elektrodialyse

Eine 30 %ige Lösung aus N-Dodeclyaminopolyolen und Isomalt (Mischung aus 6-O-(α-D-Glucopyranosyl-D-mannit -D-mannit bzw. -sorbit) (Verhältnis 2:1) wird in einer

Elektrodialyseanlage (Firma Bergholt, Typ Bel II) gegen vollentsalztes Wasser/Isopropanol (80/20; v/v) dialysiert. Bei einer Spannung von 16 V, einer Membranfläche von 360 cm² wird nach 3 h ein Konzentrat mit einem Feststoffgehalt von 8,5 % erhalten. HPLC-Analysen (high performance liquid chromatography) bestätigen, daß das Konzentrat aus 95 % N-Alkylaminopolyolen besteht.

Beispiele zu Eigenschaften hinsichtlich der Verwendung als hydrophiles Netzmittel, waschaktive Substanzen, Emulgatoren bzw. Detergentien.

### Beispiel 6

### Oberflächenspannungen und kritische Micellbildungskonzentration (CMC)

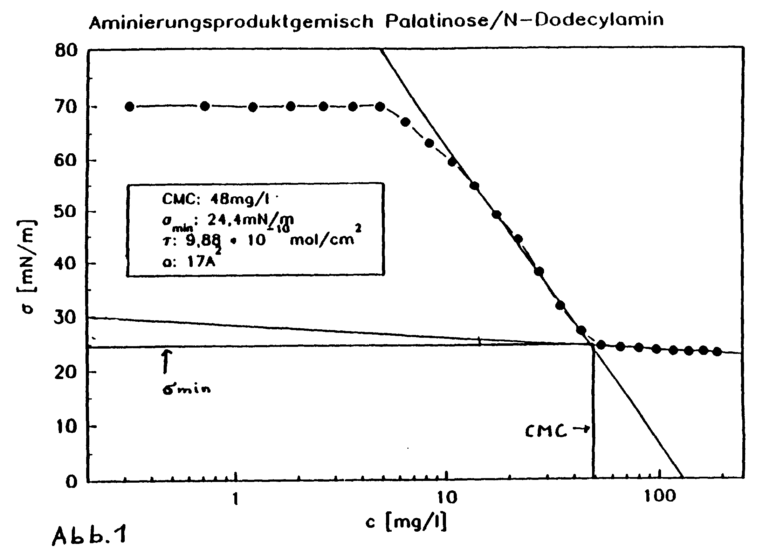

Es wurden für verschiedene Aminierungsmittel (Isomaltulose mit N-Alkylamin) die Abhängigkeiten der Oberflächenspannungen von den eingesetzten Produktkonzentrationen mit der Plattenmethode nach Wilhelmy (H. Nassenstein, "Grenzflächenforschung und Verfahrenstechnik", Chem.-Ing.-Tech., 53, S. 631-37 (1981) in Wasser bei 25 °C ermittelt (Abb. 1). Aus den konzentrationsabhängigen Oberflächenspannungsmessungen wird neben der Oberflächenspannung σ ebenfalls die kritische Micellbildungskonzentration, kurz CMC genannt, erhalten.

**Tabelle II:**

| Die folgende Tabelle II zeigt die Werte von Aminierungsgemischen mit verschiedenen N-Alkylrestkettenlängen auf der Basis von Isomaltulose, wobei die mit APG und (EO)₉ - C₁₂H₂₅ bezeichneten Gemische Vergleichsversuche darstellen. | | |
|---|---|---|
| **Tensidgemisch (Hauptkomponente)** | **σ (mN/m)** | **CMC (mg/l)** |
| 1-N-Oktylaminoisomalt | 27 | 105 |
| 1-N-Decylaminoisomalt | 27 | 61 |
| 1-N-Dodecylaminoisomalt | 24 | 48 |
| 1-N-Hexadecylaminoisomalt | 28 | 16 |
| 1-N-Cocoaminoisomalt** | 28 | 18 |
| APG (Coco) (Vergleich) | 28 | 18 |
| (EO)₉ - C₁₂H₂₅ (Vergleich) | 33,5 | 42 |

| | | |
|---|---|---|
| ** Coco: mittlere Alkylkettenlänge ca. C₁₂ | | |

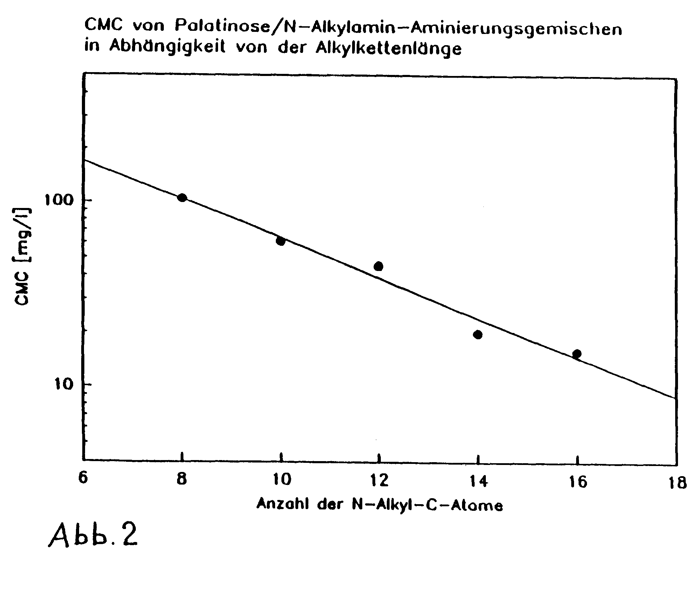

Gegenüber den sogenannten Alkylpolyglycosiden, kurz als APG bezeichnet, die ebenfalls aus einem Kohlenhydratbaustein und einem Alkylrest aufgebaut sind und Ethylenoxidtensiden (beispielhaft wurde Nonaethylenoxid-dodecylether verwendet) weisen die hier beschriebenen neuen Produkte äquivalente Oberflächenspannungen auf, zeigen hinsichtlich CMC noch niedrigere Werte (Abb. 2), so daß davon ausgegangen werden kann, daß in entsprechenden Formulierungen durch einen geringen Einsatz das gleiche Resultat erzielt werden kann.

### Beispiel 7

### Oberflächenspannungen und kritische Micellbildungskonzentrationen in Elektrolytlösungen

Obwohl die erfindungsgemäßen Verbindungen einen potentiell kationischen Charakter aufweisen, verändern sich Oberflächenspannung und CMC in Salzlösungen nur geringfügig.

**Tabelle III:**

| Die folgende Tabelle III zeigt die Oberflächenspannungen und CMC von N-Dodecylaminoisomalt und Vergleichstensiden in unterschiedlichen Salzlösungen. | | | |
|---|---|---|---|
| **Tensidgemisch (Hauptkomponente)** | **Lösungsmittel** | **σ(mN/m)** | **CMC (mg/l)** |
| 1-N-Dodecylaminoisomalt | H₂O | 27 | 48 |
| 1-N-Dodecylarninoisomalt | 0,5 M NaCl | 23 | |
| 1-N-Dodecylaminoisomalt | 0,1 M NaCl | 24 | 48 |
| 1-N-Dodecylaminoisomalt | 0,05 M NaCl | 25 | 49 |
| 1-N-Dodecylaminoisomalt | 0,01 M NaCl | 25 | 48 |
| 1-N-Dodecylaminoisomalt | 0,5 M NaCl | 24 | 38 |
| 1-N-Dodecylaminoisomalt | 0,05 M CaCl₂ | 24 | 39 |
| 1-N-Dodecylaminoisomalt | 0,01 M CaCl₂ | 25 | 43 |
| 1-N-Cocoaminoisomalt | 0,1 M NaCl | 24 | 10 |
| 1-N-Cocoaminoisomalt | 0,1 M CaCl₂ | 23 | 8 |
| APG (Vergleich) | H₂O | 28 | 24 |
| APG (Vergleich) | 0,1 M CaCl₂ | 29 | 54 |

Durch Elektrolytzugabe wird die CMC teilweise noch weiter abgesenkt. Bei anderen Tensiden wie z. B. APG's bewirkt der Elektrolyteinfluß, besonders bei zweiwertigen Kationen, eine Erhöhung der kritischen Micellbildungskonzentration.

### Beispiel 8

### Verwendung der Produkte als Softergent

Vorversuche bestätigen, daß die neuen Produkte mit anionischen Tensiden nicht zu Ausflockungen führen. Es wurden je 1 % Lösung von N-Cocoaminoisomalt und Fettalkoholethersulfat zusammengegeben und bis 90 °C erhitzt. Eine Ausfällung war auch beim Abkühlen nicht zu beobachten. In einem standardisierten Waschversuch konnte geprüft werden, daß bereits eine 1 % Zugabe eine weichmachende Wirkung auf die Wäsche ausübt.

### Beispiel 9

### Schaumvermögen hinsichtlich der Anwendung als Tensid

Das Schaumvermögen bzw. die Schaumstabilität wurde beim N-Cocoaminoisomalt als 0,1 %ige wäßrige Lösung nach DIN 53 902 vermessen. Die erzielten Werte (Ablesung nach 30 s/300 s) liegen bei diesem Produkt bei 585 ml/580 ml und liegen somit in der Größenordnung, wie sie für das kommerziell in hohem Umfang eingesetzte Anionentensid LAS (590 ml/570 ml) gefunden werden. Die Eignung als waschaktive Substanz ist somit gegeben.

## Patentansprüche

1. Mischungen
1-N-Alkylamino-1-desoxy-6-0-[α-D-glucopyranosyl]-D-sorbit und
1-N-Alkylamino-1-desoxy-6-0-[α-D-glucopyranosyl]-D-mannit,
das auch als 1-N-Alkylaminoisomalt bezeichnet die folgende allgemeine Formel I hat: 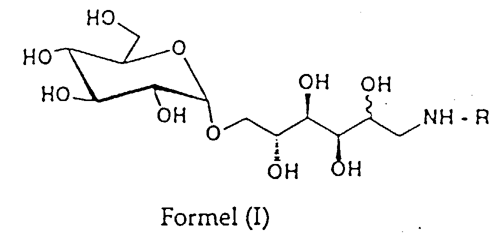 in der R eine verzweigte oder unverzweigte, gesättigte oder ungesättigte C-₈ bis C-₂₁ Alkylgruppe bedeutet,
mit 1-N-Alkyl-amino-1-desoxy-3-O-α-glucopyranosylglycerin der Formel II 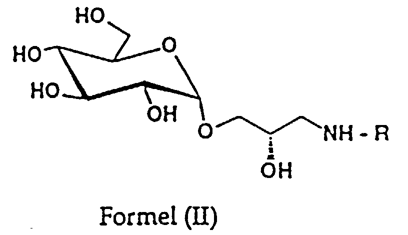 und gegebenenfalls mit 1-N-Alkylamino-1-desoxy-sorbit bzw. -mannit und 1-N-Alkylamino-1-desoxy-glycerin, wobei die Alkylgruppe die Bedeutung wie in Formel I hat.

2. Verfahren zur Herstellung eines stereoisomeren Gemisches aus
1-N-Alkylamino-1-desoxy-6-0-[α-D-glucopyranosyl]-D-sorbit und
1-N-Alkylamino-1-desoxy-6-0-[α-D-glucopyranosyl]-D-mannit,
das auch als 1-N-Alkylaminoisomalt bezeichnet die folgende allgemeine Formel I hat: 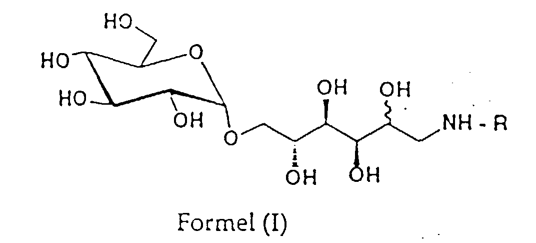 in der R eine verzweigte oder unverzweigte, gesättigte oder ungesättigte C-₈ bis C-₂₁ Alkylgruppe bedeutet,
oder aus Mischungen von 1-N-Alkylaminoisomalt der Formel I mit 1-N-Alkyl-amino-1-desoxy-3-O-α-glucopyranosylglycerin, der Formel II 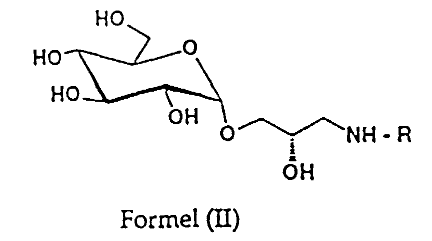 und/oder mit 1-N-Alkylamino-1-desoxy-sorbit bzw. -mannit und 1-N-Alkylamino-1-desoxy-glycerin,wobei die Alkylgruppe die Bedeutung wie in Formel I hat,
dadurch gekennzeichnet, daß Isomaltulose mit N-Alkylaminen bzw. Mischungen von N- Alkylaminen, in denen die Alkylgruppe die gleiche Bedeutung wie in Formel I hat, mit Metallkatalysatoren und Wasserstoff in wäßriger oder wäßrig-alkoholischer Lösung reduktiv aminiert wird.

3. Verfahren nach Anspruch 2, **dadurch gekennzeichnet**, daß das Rohprodukt mittels chromatographischer Methoden getrennt wird.

4. Verfahren gemäß einem der Ansprüche 2 bis 3, **dadurch gekennzeichnet**, daß ein Überschuß von 0,5 bis 5 Mol, vorzugsweise 2 bis 4 Mol, Isomaltulose gegenüber dem Alkylamin eingesetz wird.

5. Verfahren gemäß einem der Ansprüche 2 bis 4, **dadurch gekennzeichnet**, daß der Reaktionsmischung 0,5 bis 10 Gew.-% von Produkten der Formel I zugemischt werden.

6. Verfahren gemäß einem der Ansprüche 2 bis 5, bei denen die aminierten Produkte mittels Elektrodialyse von nicht aminierten Produkten abgetrennt werden.

7. Verwendung der Mischungen gemäß Anspruch 1 als hydrophile Netzmittel, Emulgatoren oder waschaktive Substanzen, insbesondere in kosmetischen bzw. technischen Reinigungsmitteln.

## Claims

1. Mixtures of 1-N-alkylamino-1-desoxy-6-0-[α-D-glucopyranosyl]-D-sorbitol and 1-N-alkylamino-1-desoxy-6-0-[α-D-glucopyranosyl]-D-mannitol, which also have the following general Formula I designated as 1-N-alkylaminoisomalt: 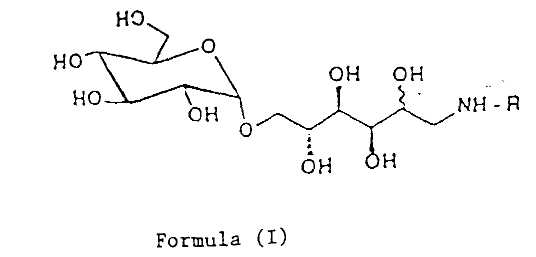 in which R means a branched or unbranched, saturated or unsaturated C-₈ to C-₂₁ alkyl group, with 1-N-alkyl-amino-1-desoxy-3-0-α-glucopyranosyl-glycerol of the Formula II 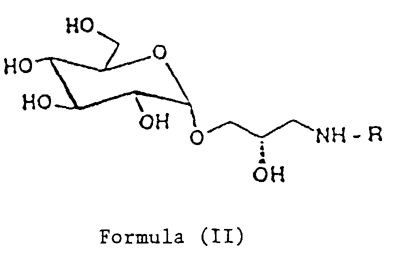 and, if appropriate, with 1-N-alkylamino-1-desoxy-sorbitol or 1-N-alkylamino-1-desoxy-mannitol and 1-N-alkylamino-1-desoxy-glycerol, with the alkyl group having the meaning as in Formula I.

2. Method for preparing a stereo-isomeric mixture from 1-N-alkylamino-1-desoxy-6-0-[α-D-glucopyranosyl]-D-sorbitol and 1-N-alkylamino-1-desoxy-6-0-[α-D-glucopyranosyl]-D-mannitol, which also has the following general Formula I designated as 1-N-alkylaminoisomalt: 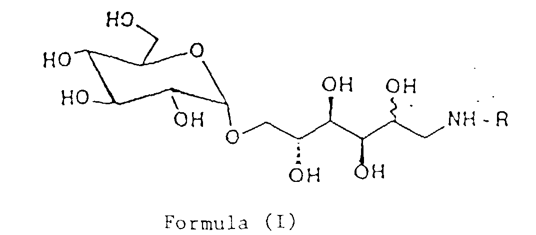 in which R means a branched or unbranched, saturated or unsaturated C-₈ to C-₂₁ alkyl group, or from mixtures of 1-N-alkylaminoisomalt of Formula I with 1-N-alkyl-amino-1-desoxy-3-0-α-glucopyranosyl-glycerol of Formula II, 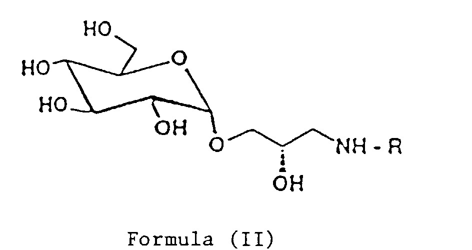 and/or with 1-N-alkylamino-1-desoxy-sorbitol or 1-N-alkylamino-1-desoxy-mannitol and 1-N-alkylamino-1-desoxy-glycerol, with the alkyl group having the meaning as in Formula I, characterised in that isomaltulose having N-alkylamines or mixtures of N-alkylamines, in which the alkyl group has the same meaning as in Formula I, is reductively aminated with metal catalysts and hydrogen in aqueous or aqueousalcoholic solution.

3. Method according to claim 2, characterised in that the raw product is separated by means of chromatographic methods.

4. Method in accordance with one of the claims 2 to 3, characterised in that an excess of 0.5 to 5 mole, preferably 2 to 4 mole, of isomaltulose with respect to the alkylamine is used.

5. Method in accordance with one of the claims 2 to 4, characterised in that 0.5 to 10 % by weight of products of Formula I are added to the reaction mixture.

6. Method in accordance with one of the claims 2 to 5, in which the aminated products are dissociated from non-aminated products by means of electrodialysis.

7. Use of the mixtures in accordance with claim 1 as hydrophilic wetting agents, emulsifiers or surface-active substances, in particular in cosmetic or technical cleansing agents.

## Revendications

1. Mélanges constitués de
1-N-alkylamino-1-désoxy-6-0-[α-D-glucopyrasonyl]-D-sorbite et de
1-N-alkylamino-1-désoxy-6-0-[α-D-glucopyrasonyl]-D-mannitol, désigné également par l'appellation 1-N-alkylaminoisomalt et ayant la formule générale I : 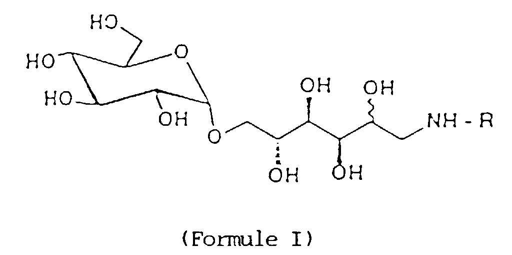 dans laquelle R est un groupe alkyle ramifié ou non ramifié, saturé ou insaturé, en C₈ à C₂₁,
avec de la 1-N-alkyl-amino-1-désoxy-3-0-a-glucopyrasonyl-glycérine de formule II 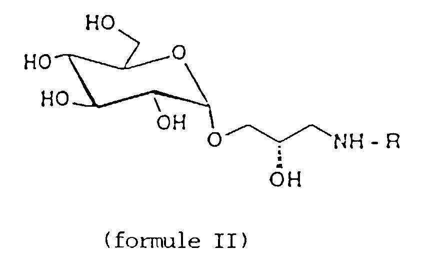 et, le cas échéant, avec de la 1-N-alkylamino-1-désoxy-sorbite, respectivement mannitol et de la 1-N-alkylanino-1-désoxy-glycérine, le groupe alkyle ayant la même signification que dans la formule I.

2. Procédé de fabrication d'un mélange stéréoisomère constitué de 1-N-alkylamino-1-désoxy-6-0-[α-D-glucopyrasonyl]-D-sorbite et de 1-N-alkylamino-1-désoxy-6-0-[α-D-glucopyrasonyl]-D-mannitol, désigné également par l'appellation 1-N-alkylaminoisomalt et ayant la formule générale I : 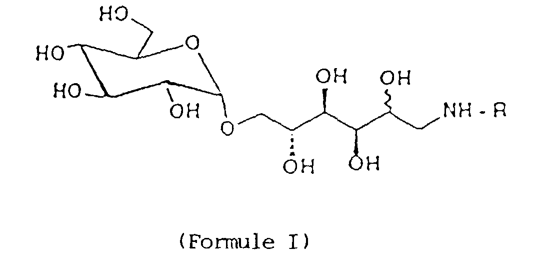 dans laquelle R est un groupe alkyle ramifié ou non ramifié, saturé ou insaturé, en C₈ à C₂₁,
ou de mélanges de 1-N-alkylaminoisomalt de formule I avec de la 1-N-alkyl-amino-1-désoxy-3-0-a-glucopyrasonylglycérine de formule II 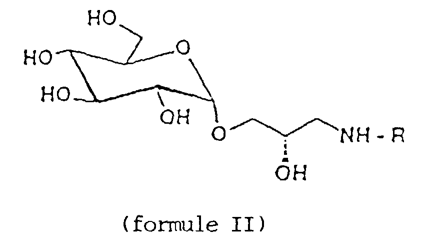 et/ou avec de la 1-N-alkylamino-1-désoxy-sorbite, respectivement mannitol et de la 1-N-alkylamino-1-désoxy-glycérine, le groupe alkyle ayant la même signification que dans la formule I,
caractérisé en ce que l'isomaltulose est aminée par voie réductive avec des N-alkylamines, respectivement des mélanges de N-alkylamines, dans lesquelles le groupe alkyle a la même signification que dans la formule I, avec des métaux catalyseurs et de l'hydrogène dans une solution aqueuse ou alcoolo-aqueuse.

3. Procédé selon la revendication 2, caractérisé en ce que le produit brut est séparé à l'aide de méthodes chromatographiques.

4. Procédé selon la revendication 2 ou 3, caractérisé en ce qu'est utilisé un excès de 0,5 à 5 moles, de préférence de 2 à 4 moles d'isomaltulose, par rapport à l'alkylamine.

5. Procédé selon l'une des revendications 2 à 4, caractérisé en ce qu'au mélange de réaction est ajouté de 0,5 à 10 % en poids de produits de formule I.

6. Procédé selon l'une des revendications 2 à 5, dans lesquels les produits aminés sont séparés des produits non aminés, à l'aide d'une électrodialyse.

7. Utilisation des mélanges selon la revendication 1 comme réticulants hydrophiles, émulsifiants ou substances présentant une activité détergente, en particulier dans des agents nettoyants cosmétiques ou techniques.
